# EUROPÄISCHE PATENTANMELDUNG

(11) **EP 4 681 766 A1**
(43) Veröffentlichungstag der Anmeldung: **21.01.2026**
(21) Anmeldenummer: 25189524.9
(22) Anmeldetag: 15.07.2025
(51) Int. Cl.: A61M 29/00, A61M 25/00, A61M 25/06

(54) **DILATATOR ZUR VERWENDUNG BEI EINER KATHETERANLAGE**

(30) Priorität: 18.07.2024 DE 102024120392
(71) Anmelder: B. Braun Melsungen AG, 34212 Melsungen (DE)
(72) Erfinder: Wildhagen, Jens, 30659 Hannover (DE)
(74) Vertreter: Patentanwälte Ruff, Wilhelm, Beier, Dauster & Partner mbB

(57) **Zusammenfassung**

Die Erfindung betrifft ein medizinisches Gerät zur Verwendung bei einer Katheteranlage, aufweisend eine Längsachse (L), einen Kapillarabschnitt (2), der koaxial zu der Längsachse (L) zwischen einem proximalen Kapillarende (21) und einem distalen Kapillarende (22) längserstreckt ist, und der eine biegenachgiebige Gestaltung und einen Kapillaraußendurchmesser (23) aufweist, einen Dilatatorabschnitt (3), der koaxial zu der Längsachse (L) zwischen einem proximalen Dilatatorende (31) und einem distalen Dilatatorende (32), das axial an das proximale Kapillarende (21) angrenzt, längserstreckt ist, und der eine formstabile Gestaltung sowie einen Dilatatoraußendurchmesser (33) aufweist, der wenigstens abschnittsweise größer ist als der Kapillaraußendurchmesser (23), und ein Lumen (4), das koaxial zu der Längsachse (L) durchgängig zwischen dem proximalen Dilatatorende (31) und dem distalen Kapillarende (22) durch den Dilatatorabschnitt (3) und den Kapillarabschnitt (2) längserstreckt und zur Aufnahme einer Kanüle (7) eingerichtet ist.

## Beschreibung

Die Erfindung betrifft ein medizinisches Gerät zur Verwendung bei einer Katheteranlage.

Es ist bekannt, dass zum Anlegen eines zentralvenösen Katheters mehrere Schritte notwendig sind. Bei einer als Seldinger-Technik bekannten Vorgehensweise wird unter Verwendung einer Kanüle zunächst ein venöser Zugang geschaffen. Hiernach wird durch die angelegte Kanüle ein Führungsdraht in die betreffende Vene eingeführt. Nach dem Einführen des Führungsdrahts wird die Kanüle proximal über den Führungsdraht abgezogen und entfernt. Hiernach wird der in die Vene reichende Einstichkanal der Kanüle unter Verwendung eines Dilatators geweitet. Hierfür wird der Dilatator distal über den Führungsdraht aufgeschoben und in den Einstichkanal gedrückt, wodurch dieser geweitet wird. Nachdem der Einstichkanal ausreichend geweitet ist, wird der Dilatator proximal über den Führungsdraht abgezogen und entfernt. Hiernach erfolgt die eigentliche Katheteranlage, bei welcher der zentralvenöse Katheter distal über den Führungsdraht aufgeschoben, in den geweiteten Einstichkanal eingeführt und längs der Vene bis zu einer Bestimmungsposition vorgeschoben wird. Abschließend wird der Führungsdraht proximal aus dem Katheter herausgezogen und entfernt und das distale Katheterende wird außerhalb der Vene auf der Haut des Patienten befestigt.

Aufgabe der Erfindung ist es, ein medizinisches Gerät bereitzustellen, das eine vereinfachte Katheteranlage ermöglicht und eine verbesserte Patientensicherheit bietet.

Diese Aufgabe wird durch das Bereitstellen eines medizinischen Geräts mit den Merkmalen des Anspruchs 1 gelöst. Vorteilhafte Weiterbildungen sind in den Unteransprüchen angegeben. Der Wortlaut der Ansprüche wird durch Bezugnahme zum Gegenstand der Beschreibung gemacht.

Das erfindungsgemäße medizinische Gerät weist eine Längsachse, einen Kapillarabschnitt, einen Dilatatorabschnitt und ein Lumen auf. Der Kapillarabschnitt ist koaxial zu der Längsachse zwischen einem proximalen Kapillarende und einem distalen Kapillarende längserstreckt. Der Kapillarabschnitt weist eine biegenachgiebige Gestaltung und einen Kapillaraußendurchmesser auf. Der Dilatatorabschnitt ist koaxial zu der Längsachse zwischen einem proximalen Dilatatorende und einem distalen Dilatatorende längserstreckt. Das distale Dilatatorende grenzt axial an das proximale Kapillarende an. Der Dilatatorabschnitt weist eine formstabile Gestaltung und einen Dilatatoraußendurchmesser auf. Der Dilatatoraußendurchmesser ist wenigstens abschnittsweise größer als der Kapillaraußendurchmesser. Das Lumen ist koaxial zu der Längsachse durch den Dilatatorabschnitt und den Kapillarabschnitt längserstreckt. Das Lumen ist durchgängig zwischen dem proximalen Dilatatorende und dem distalen Kapillarende längserstreckt. Das Lumen ist zur Aufnahme einer Kanüle eingerichtet.

Die Erfindung geht von der Erkenntnis aus, dass die aus dem Stand der Technik bekannte Seldinger-Technik die Verwendung mehrerer unterschiedlicher medizinischer Geräte erfordert. Diese unterschiedlichen medizinischen Geräte müssen zunächst bereitgestellt und während der Katheteranlage von dem ausführenden medizinischen Personal nacheinander verwendet werden. Dies ist zeitaufwändig und fehleranfällig. Die Erfindung geht weiter von der Erkenntnis aus, dass es beim Einführen des Führungsdrahts durch die angelegte Kanüle zu einer Verletzung der dem Einstichkanal gegenüberliegenden Venenwand kommen kann. Solche Verletzungen können durch unbeabsichtigte Bewegungen der angelegten Kanüle oder den Führungsdraht selbst verursacht werden. Das erfindungsgemäße medizinische Gerät wirkt den geschilderten Nachteilen entgegen. Dabei sieht die Verwendung des erfindungsgemäßen medizinischen Geräts vor, dass der Kapillarabschnitt durch einen Einstichkanal in die betreffende Vene eingeführt wird. Der Einstichkanal kann durch eine Kanüle hergestellt sein, die beispielsweise in das Lumen des medizinischen Geräts axial eingeführt ist und deren distale Kanülenspitze über das distale Kapillarende hinausragt. Dabei werden die Kanüle und der Kapillarabschnitt gemeinsam in das Körpergewebe eingestochen und in die Vene eingeschoben. Die besagte Kanüle kann hiernach proximal aus dem Lumen herausgezogen und entfernt werden. Hiernach wird der Führungsdraht durch das Lumen des medizinischen Geräts und damit durch den Dilatatorabschnitt und den Kapillarabschnitt in die Vene eingeschoben. Durch die biegenachgiebige Gestaltung des Kapillarabschnitts wird eine Verletzung der Venenwand bei unbeabsichtigten Bewegungen des medizinischen Geräts vorgebeugt. Zudem erlaubt der biegenachgiebige Kapillarabschnitt eine verbesserte Führung des Führungsdrahts in Längsrichtung der Vene, was Verletzungen durch den Führungsdraht vorbeugt. Nach dem Einführen des Führungsdrahts kann der Einstichkanal unter Einwirkung des Dilatatorabschnitts geweitet werden. Hierfür wird das medizinische Gerät distal vorgeschoben, bis der Dilatatorabschnitt in den Einstichkanal eindringt und diesen weitet. Durch die biegenachgiebige Gestaltung des Kapillarabschnitts wird auch bei diesem distalen Vorschieben des medizinischen Geräts einer Verletzung der Venenwand vorgebeugt. Nach dem Weiten des Einstichkanals kann das medizinische Gerät proximal über den Führungsdraht abgezogen und entfernt werden. Hiernach kann der zentralvenöse Katheter über den Führungsdraht aufgeschoben, in den geweiteten Einstichkanal eingeführt und entlang der Vene bis zu einer Bestimmungsposition vorgeschoben werden. Vor dem Hintergrund der geschilderten Verwendungsweise des erfindungsgemäßen medizinischen Geräts wird deutlich, dass die Erfindung eine maßgebliche Steigerung der Effizienz bei der Katheteranlage ermöglicht. Zudem wird die Patientensicherheit verbessert, indem Verletzungen der Venenwand vorgebeugt werden. Der Kapillarabschnitt ist biegenachgiebig, wohingegen der Dilatatorabschnitt formstabil ist. Bei einer Ausgestaltung sind der Kapillarabschnitt und der Dilatatorabschnitt aus unterschiedlichen Werkstoffen mit unterschiedlichen Elastizitätsmoduln gefertigt, vorzugsweise aus unterschiedlichen Kunststoffen. Bei einer Ausgestaltung ist der Kapillarabschnitt weichelastisch. Bei einer Ausgestaltung ist der Kapillarabschnitt durch einen Schlauchabschnitt gebildet, der insbesondere auch als Kurzkatheter bezeichnet werden kann. Vorzugsweise ist der Kapillaraußendurchmesser über einer Länge des Kapillarabschnitts konstant und/oder unveränderlich. Weiter vorzugsweise ist der Kapillaraußendurchmesser rund, im Speziellen kreisrund. Bei einer Ausgestaltung ist der Dilatatoraußendurchmesser über eine Länge des Dilatatorabschnitts unveränderlich und/oder konstant. Bei einer bevorzugten Ausgestaltung ist der Dilatatoraußendurchmesser über der Länge des Dilatatorabschnitts veränderlich. Vorzugsweise ist der Dilatatoraußendurchmesser rund, im Speziellen kreisrund. Das distale Dilatatorende und das proximale Kapillarende grenzen axial unmittelbar aneinander an. Mit anderen Worten: Das proximale Kapillarende geht in das distale Dilatatorende über und umgekehrt. Dieser Übergang ist vorzugsweise radial stufenlos.

Das erfindungsgemäße medizinische Gerät eignet sich in besonderem Maße zur Verwendung bei der Anlage eines zentralvenösen Katheters. Das erfindungsgemäße medizinische Gerät kann aber auch bei der Anlage anderer Katheter vorteilhaft zur Verwendung kommen.

In Ausgestaltung der Erfindung ist der Dilatatoraußendurchmesser über der Länge des Dilatatorabschnitts veränderlich und nimmt ausgehend von dem distalen Dilatatorende in proximaler Richtung zu. Diese Zunahme ist vorzugsweise kontinuierlich. Durch den veränderlichen und in distaler Richtung zunehmenden Dilatatoraußendurchmesser ist gewährleistet, dass der Dilatatorabschnitt mit geringem Kraftaufwand in den Einstichkanal gedrückt werden kann. Zudem wird einer übermäßigen Beanspruchung des Körpergewebes im Bereich des Einstichkanals entgegengewirkt.

In weiterer Ausgestaltung der Erfindung entspricht der Dilatatoraußendurchmesser im Bereich des distalen Dilatatorendes dem Kapillaraußendurchmesser und umgekehrt. Bei dieser Ausgestaltung ist der Übergang zwischen Kapillarabschnitt und Dilatatorabschnitt radial stufenlos. Dies jedenfalls in praktischer Hinsicht. Durch den stufenlosen Übergang zwischen dem proximalen Kapillarende und dem distalen Dilatatorende wird vermieden, dass das distale Dilatatorende beim Weiten des Einstichkanals stumpf an den Rand des Einstichkanals anstößt. Vielmehr wird durch die stufenlose Gestaltung ein besonders leichtgängiges Einführen des distalen Dilatatorendes in den Einstichkanal gewährleistet.

In weiterer Ausgestaltung der Erfindung weist der Dilatatorabschnitt einen Kegelbereich und einen Zylinderbereich auf, wobei der Kegelbereich ausgehend von dem distalen Dilatatorende mit zunehmendem Dilatatoraußendurchmesser proximal längserstreckt ist und in den Zylinderbereich übergeht, wobei der Dilatatoraußendurchmesser in dem Zylinderbereich axial unveränderlich und/oder maximal ist. Der Kegelbereich ermöglicht ein besonders einfaches und leichtgängiges Eindringen in den Einstichkanal. Zu diesem Zweck weist der Kegelbereich den ausgehend von dem distalen Dilatatorende zunehmenden Dilatatoraußendurchmesser auf. Der sich proximal unmittelbar an den Kegelbereich anschließende Zylinderbereich dient dem eigentlichen Aufweiten des Einstichkanals. Zu diesem Zweck kann der Zylinderbereich einmalig oder auch mehrfach innerhalb des Einstichkanal axial hin- und herbewegt werden. Im Unterschied zu dem Kegelbereich ist der Dilatatoraußendurchmesser in dem Zylinderbereich axial unveränderlich.

In weiterer Ausgestaltung der Erfindung beträgt eine Länge des Kapillarabschnitts zwischen 80 % und 120 %, bevorzugt zwischen 90 % und 110 %, besonders bevorzugt zwischen 95 % und 105 %, einer Länge des Dilatatorabschnitts. Mit anderen Worten: Der Kapillarabschnitt und der Dilatatorabschnitt sind in etwa gleich lang. Dabei haben sich die vorstehenden Wertebereiche für das Verhältnis zwischen der Länge des Kapillarabschnitts und der Länge des Dilatatorabschnitts als besonders vorteilhaft erwiesen.

In weiterer Ausgestaltung der Erfindung beträgt der Dilatatoraußendurchmesser wenigstens 120 %, bevorzugt wenigstens 150 %, besonders bevorzugt wenigstens 200 %, des Kapillaraußendurchmessers. Hierdurch ist gewährleistet, dass der Einstichkanal ausreichend geweitet werden kann.

In weiterer Ausgestaltung der Erfindung sind das proximale Kapillarende und das distale Dilatatorende mittels einer Fügeverbindung verbunden. Bei einer Ausgestaltung ist die Fügeverbindung eine stoffschlüssige Verbindung, beispielsweise eine Klebeverbindung oder eine Schweißverbindung. Alternativ oder zusätzlich sind der Kapillarabschnitt und der Dilatatorabschnitt axial zusammengesteckt.

In weiterer Ausgestaltung der Erfindung weist das medizinische Gerät zudem einen Griffabschnitt auf. Der Griffabschnitt ist mit dem proximalen Dilatatorende verbunden und zum manuellen Greifen durch einen Benutzer eingerichtet. Der Griffabschnitt erleichtert die Verwendung des medizinischen Geräts, insbesondere beim Weiten des Einstichkanals. Vorzugsweise weist der Griffabschnitt einen Außendurchmesser auf, der größer ist als der (maximale) Dilatatoraußendurchmesser.

In weiterer Ausgestaltung der Erfindung weist das medizinische Gerät zudem einen Fluidkonnektor auf. Der Fluidkonnektor mündet distal in das Lumen und ist wenigstens mittelbar mit dem proximalen Dilatatorende verbunden. Sofern das medizinische Gerät einen Griffabschnitt gemäß der vorhergehenden Ausgestaltung aufweist, ist der Fluidkonnektor vorzugsweise an einem proximalen Ende des Griffabschnitts angeordnet. Der Fluidkonnektor ist zur Verbindung mit einer fluidführenden Komponente eingerichtet, beispielsweise einer Spritze oder einem Schlauch. Bei einer Ausgestaltung ist der Fluidkonnektor ein Luer-Konnektor, beispielsweise ein Luer-Lock-Konnektor oder ein Luer-Slip-Konnektor. Es sind auch andere Konnektortypen denkbar und möglich.

In weiterer Ausgestaltung der Erfindung weist das medizinische Gerät zudem eine Kanüle auf, die axial in das Lumen eingeführt oder einführbar ist und deren distale Kanülenspitze in eingeführtem Zustand über das distale Kapillarende hinausragt. Zum Ausbilden des Einstichkanals kann die distale Kanülenspitze in das Körpergewebe des Patienten eingestochen werden. Der Kapillarabschnitt ist radial auf der Kanüle abgestützt und wird gemeinsam mit dieser in das Körpergewebe und damit die Vene eingeführt. Nach dem Ausbilden des Einstichkanals kann die Kanüle distal aus dem Lumen herausgezogen und entfernt werden, wobei der Kapillarabschnitt in der Vene verbleibt.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnung dargestellt ist.

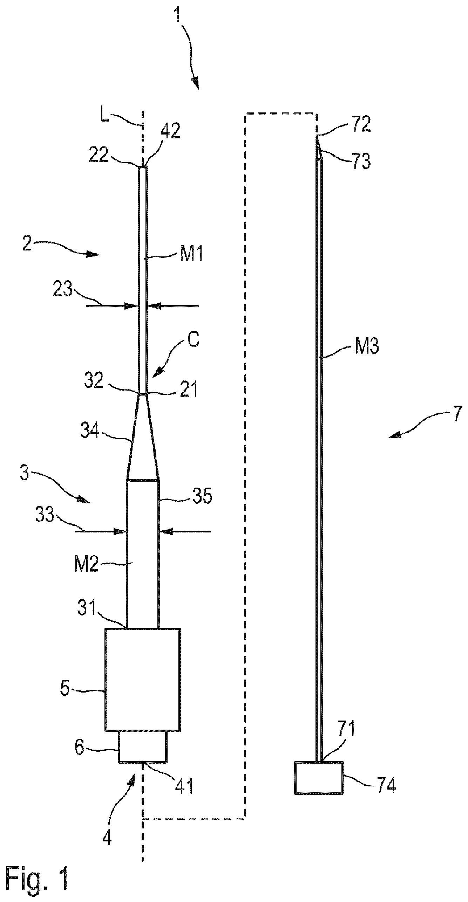

- Fig. 1: zeigt in schematischer Seitenansicht eine Ausführungsform eines erfindungsgemäßen medizinischen Geräts.

Gemäß Fig. 1 ist ein medizinisches Gerät 1 zur Verwendung bei einer Katheteranlage vorgesehen. Das medizinische Gerät 1 ermöglicht eine effiziente und besonders sichere Anlage des betreffenden Katheters und ist im Speziellen geeignet für zentralvenöse Katheter.

Das medizinische Gerät 1 weist eine Längsachse L, einen Kapillarabschnitt 2, einen Dilatatorabschnitt 3 und ein Lumen 4 auf.

Bei der gezeigten Ausführungsform weist das medizinische Gerät 1 zudem einen Griffabschnitt 5, einen Fluidkonnektor 6 und eine Kanüle 7 auf. Der Griffabschnitt 5, der Fluidkonnektor 6 und die Kanüle 7 sind jeweils optional und folglich nicht bei allen Ausführungsformen vorhanden.

Der Kapillarabschnitt 2 ist koaxial zu der Längsachse L längserstreckt. Der Kapillarabschnitt 2 weist ein proximales Kapillarende 21 und ein distales Kapillarende 22 auf. Der Kapillarabschnitt 2 ist zwischen dem proximalen Kapillarende 21 und dem distalen Kapillarende 22 längserstreckt. Der Kapillarabschnitt 2 weist eine biegenachgiebige Gestaltung auf. Der Kapillarabschnitt 2 weist einen Kapillaraußendurchmesser 23 auf.

Der Dilatatorabschnitt 3 ist koaxial zu der Längsachse L und damit auch koaxial zu dem Kapillarabschnitt 2 längserstreckt. Der Dilatatorabschnitt 3 weist ein proximales Dilatatorende 31 und ein distales Dilatatorende 32 auf. Der Kapillarabschnitt 3 ist zwischen dem proximalen Dilatatorende 31 und dem distalen Dilatatorende 32 längserstreckt. Das distale Dilatatorende 32 und das proximale Kapillarende 21 grenzen axial aneinander an. Der Dilatatorabschnitt 3 weist eine formstabile Gestaltung auf. Der Dilatatorabschnitt 3 weist einen Dilatatoraußendurchmesser 33 auf, der wenigstens abschnittsweise größer ist als der Kapillaraußendurchmesser 23.

Das Lumen 4 ist koaxial zu der Längsachse L und damit auch koaxial zu dem Kapillarabschnitt 2 und dem Dilatatorabschnitt 3 längserstreckt. Das Lumen 4 erstreckt sich durchgängig zwischen dem proximalen Dilatatorende 31 und dem distalen Kapillarende 22 durch den Dilatatorabschnitt 3 und dem Kapillarabschnitt 2. Das Lumen 4 ist zur Aufnahme der Kanüle 7 eingerichtet. Das Lumen 4 weist eine proximale Öffnung 41 und eine distale Öffnung 42 auf. Das Lumen 4 ist zwischen der proximalen Öffnung 41 und der distalen Öffnung 42 durchgängig längserstreckt. Bei der gezeigten Ausführungsform ist die proximale Öffnung 41 an dem Fluidkonnektor 6 ausgebildet. Die distale Öffnung 42 ist an dem distalen Kapillarende 22 ausgebildet.

Bei Ausführungsformen ohne Fluidkonnektor kann die proximale Öffnung an dem proximalen Dilatatorende oder einem proximalen Ende des Griffabschnitts ausgebildet sein.

Bei Ausführungsformen ohne Fluidkonnektor und ohne Griffabschnitt kann die proximale Öffnung an dem proximalen Dilatatorende ausgebildet sein.

Die biegenachgiebige Gestaltung des Kapillarabschnitts 2 und die formstabile Gestaltung des Dilatatorabschnitts 3 können über eine entsprechende Materialwahl erreicht werden. Bei der gezeigten Ausführungsform ist der Kapillarabschnitt 2 aus einem ersten Material M1 gefertigt und der Dilatatorabschnitt 3 ist aus einem zweiten Material M2 gefertigt. Das erste Material M1 ist vorliegend ein elastomerer Kunststoff, insbesondere mit weichelastischen Eigenschaften. Das zweite Material M2 ist vorliegend ein Hartkunststoff. Bei einer in den Figuren nicht gezeigten Ausführungsform ist der Dilatatorabschnitt aus Metall gefertigt.

Bei der gezeigten Ausführungsform ist der Kapillaraußendurchmesser 23 über der Länge des Kapillarabschnitts 2 unveränderlich. Mit anderen Worten ist der Kapillaraußendurchmesser 23 konstant. Vorliegend weist der Kapillarabschnitt 2 einen runden, im Speziellen kreisrunden, Hohlquerschnitt auf.

Bei der gezeigten Ausführungsform ist der Dilatatoraußendurchmesser 33 über der Länge des Dilatatorabschnitts 3 veränderlich. Dabei nimmt der Dilatatoraußendurchmesser 33 ausgehend von dem distalen Dilatatorende 32 in proximaler Richtung zu. Diese Zunahme erstreckt sich vorliegend nicht etwa über die gesamte Länge des Dilatatorabschnitts 3, sondern lediglich bereichsweise.

Bei der gezeigten Ausführungsform entspricht der Dilatatoraußendurchmesser 33 an dem distalen Dilatatorende 32 wenigstens im Wesentlichen dem Kapillaraußendurchmesser 23 an dem proximalen Kapillarende 21 und umgekehrt. Wenigstens im Wesentlichen meint, dass die besagten Außendurchmesser ein identisches Nennmaß aufweisen, wobei aber toleranzbedingte Abweichungen möglich sind. Infolge der einander entsprechenden Außendurchmesser 23, 33 an dem distalen Dilatatorende 32 und dem proximalen Kapillarende 21 ist ein axialer Übergang zwischen dem proximalen Kapillarende 21 und dem distalen Dilatatorende 32 in radialer Richtung stufenlos.

Bei der gezeigten Ausführungsform weist der Dilatatorabschnitt einen Kegelbereich 34 und einen Zylinderbereich 35 auf.

Der Kegelbereich 34 weist das distale Dilatatorende 32 auf. Der Kegelbereich 34 ist ausgehend von dem distalen Dilatatorende 32 mit zunehmendem Dilatatoraußendurchmesser 33 proximal längserstreckt und geht in den Zylinderbereich 35 über.

Der Zylinderbereich 35 weist das proximale Dilatatorende 31 auf. Der Zylinderbereich 35 ist zwischen dem proximalen Dilatatorende 31 und dem Kegelbereich 34 längserstreckt. Der Dilatatoraußendurchmesser 33 ist in dem Zylinderbereich 35 axial unveränderlich und maximal. Demgegenüber ist der Dilatatoraußendurchmesser 33 an dem distalen Dilatatorende 32 minimal.

Der Dilatatoraußendurchmesser 33 ist in Übereinstimmung mit dem Kapillaraußendurchmesser 33 rund, im Speziellen kreisrund.

Bei der gezeigten Ausführungsform sind der Kapillarabschnitt 2 und der Dilatatorabschnitt 3 in etwa gleich lang. Bei in den Figuren nicht gezeigten Ausführungsformen beträgt die Länge des Kapillarabschnitts zwischen 80 % und 120 %, bevorzugt zwischen 90 % und 110 %, besonders bevorzugt zwischen 95 % und 105 %, der Länge des Dilatatorabschnitts.

Der (maximale) Dilatatoraußendurchmesser 33 beträgt wenigstens 120 %, bevorzugt wenigstens 150 %, besonders bevorzugt wenigstens 200 %, des Kapillaraußendurchmessers 23.

Bei der gezeigten Ausführungsform ist der Kapillarabschnitt 2 an dem Dilatatorabschnitt 3 angefügt. Zu diesem Zweck ist eine Fügeverbindung C vorhanden, mittels derer das proximale Kapillarende 21 und das distale Dilatatorende 32 miteinander zusammengefügt sind. Bei einer Ausführungsform ist die Fügeverbindung eine Klebeverbindung. Bei einer weiteren Ausführungsform ist die Fügeverbindung eine Schweißverbindung. Alternativ oder zusätzlich können der Kapillarabschnitt 2 und der Dilatatorabschnitt 3 axial zusammengesteckt sein.

Der optionale Griffabschnitt 5 ist bei der gezeigten Ausführungsform mit dem proximalen Dilatatorende 31 verbunden. Bei der besagten Verbindung zwischen dem Dilatatorabschnitt 3 und dem Griffabschnitt 5 kann es sich um eine einstückige Verbindung oder eine für den vorliegenden Zweck geeignete Fügeverbindung handeln. Der Griffabschnitt 5 ist zum manuellen Greifen durch einen Benutzer eingerichtet und erlaubt eine vereinfachte manuelle Handhabung des medizinischen Geräts 1.

Der optionale Fluidkonnektor 6 ist zur Verbindung mit einer fluidführenden Komponente eingerichtet, beispielsweise mit einer Spritze oder dergleichen. Der Fluidkonnektor 6 kann auf jede für den genannten Zweck geeignete Weise gestaltet sein, beispielsweise als Luer-Konnektor. Es sind aber auch andere Arten der Gestaltung des Fluidkonnektors denkbar und möglich. Bei der gezeigten Ausführungsform ist der Fluidkonnektor 6 mit dem Griffabschnitt 5 verbunden, im Speziellen mit einem proximalen Ende (ohne Bezugszeichen) des Griffabschnitts 5. Diese Verbindung kann einstückig oder mittels einer Fügeverbindung ausgebildet sein.

Die optionale Kanüle 7 ist axial in das Lumen 4 einführbar. In eingeführtem Zustand ragt eine distale Kanülenspitze 72 über das distale Kapillarende 22 hinaus.

Die Kanüle 7 ist auf eine der Fachperson bekannte Weise gestaltet und kann auch als Hohlnadel bezeichnet werden. Dementsprechend ist die Kanüle 7 zwischen einem proximalen Kanülenende 71 und der bereits erwähnten distalen Kanülenspitze 72 gerade längserstreckt und weist im Bereich der distalen Kanülenspitze 72 ein angeschliffenes Kanülenauge 73 auf. Die Kanüle 7 ist aus einem dritten Material M3 gefertigt, bei dem es sich vorliegend um einen für medizinische Anwendungen geeigneten Stahl handelt. Weiter weist die Kanüle 7 vorliegend einen Handhabungsabschnitt 74 auf, der an das proximale Kanülenende 71 angefügt ist. Der Handhabungsabschnitt 74 stößt in eingeführtem Zustand der Kanüle 7 axial an den Fluidkonnektor 6 an. Der Handhabungsabschnitt 74 kann einen (weiteren) Fluidkonnektor aufweisen oder als ein solcher gestaltet sein.

Das medizinische Gerät 1 kann bei der Anlage eines zentralvenösen Katheters wie folgt verwendet werden.

Sofern noch nicht geschehen, wird zunächst die Kanüle 7 in das Lumen 4 eingeführt. In eingeführtem Zustand stützt die Kanüle 7 den für sich allein genommen biegenachgiebigen Kapillarabschnitt 2 in radialer Richtung. Mit anderen Worten: Der Kapillarabschnitt 2 wird durch die eingeführte Kanüle 7 stabilisiert und ist in eingeführtem Zustand der Kanüle 7 nicht biegenachgiebig.

Das medizinische Gerät 1 wird mit der distalen Kanülenspitze 72 voran an eine zu punktierende Vene des betreffenden Patienten herangeführt. Die Vene wird mit der distalen Kanülenspitze 72 punktiert, indem das medizinische Gerät 1 distal vorgeschoben wird. Beim Einschieben oder Einstechen bildet die Kanüle 7 zusammen mit dem auf ihr abgestützten Kapillarabschnitt 2 einen Einstichkanal im Körpergewebe des Patienten. Dieser Einstichkanal reicht bis in die besagte Vene. Nachdem der venöse Zugang auf diese Weise hergestellt ist, kann die Kanüle 7 in proximaler Richtung aus dem Lumen 4 herausgezogen und entfernt werden. Das medizinische Gerät 1 kann hiernach (ohne die entfernte Kanüle 7) weiter distal vorgeschoben werden, wobei der Kapillarabschnitt 2 weiter in die Vene eingeführt wird, beispielsweise bis das distale Dilatatorende 32 an dem Einstichkanal anliegt.

Hiernach wird ein Führungsdraht in distaler Richtung durch das Lumen 4 und somit durch den Dilatatorabschnitt 3 und den Kapillarabschnitt 2 bis in die betreffende Vene vorgeschoben.

Hiernach oder alternativ auch bereits vor dem Einführen des Führungsdrahts wird der Einstichkanal unter Einwirkung des Dilatatorabschnitts 3 geweitet. Dieses Aufweiten dient einem vereinfachten Einbringen des eigentlichen zentralvenösen Katheters. Zum Weiten des Einstichkanals wird das medizinische Gerät 1 weiter distal durch den Einstichkanal vorgeschoben, wobei der im Vergleich zu dem Kapillaraußendurchmesser 23 größere Dilatatoraußendurchmesser 33 den Einstichkanal radial nach außen drückt und auf diese Weise weitet.

Nach dem Weiten des Einstichkanals wird das medizinische Gerät 1 in proximaler Richtung von dem Führungsdraht abgezogen und entfernt. Hiernach wird der zentralvenöse Katheter in proximaler Richtung über den Führungsdraht aufgeschoben, durch den geweiteten Einstichkanal in die Vene eingeführt und entlang der Vene bis zu seiner Bestimmungsposition vorgeschoben.

## Patentansprüche

1. Medizinisches Gerät (1) zur Verwendung bei einer Katheteranlage, aufweisend
eine Längsachse (L),
einen Kapillarabschnitt (2), der koaxial zu der Längsachse (L) zwischen einem proximalen Kapillarende (21) und einem distalen Kapillarende (22) längserstreckt ist, und der eine biegenachgiebige Gestaltung und einen Kapillaraußendurchmesser (23) aufweist,
einen Dilatatorabschnitt (3), der koaxial zu der Längsachse (L) zwischen einem proximalen Dilatatorende (31) und einem distalen Dilatatorende (32), das axial an das proximale Kapillarende (21) angrenzt, längserstreckt ist, und der eine formstabile Gestaltung sowie einen Dilatatoraußendurchmesser (33) aufweist, der wenigstens abschnittsweise größer ist als der Kapillaraußendurchmesser (23), und
ein Lumen (4), das koaxial zu der Längsachse (L) durchgängig zwischen dem proximalen Dilatatorende (31) und dem distalen Kapillarende (22) durch den Dilatatorabschnitt (3) und den Kapillarabschnitt (2) längserstreckt und zur Aufnahme einer Kanüle (7) eingerichtet ist.

2. Medizinisches Gerät (1) nach Anspruch 1, wobei der Dilatatoraußendurchmesser (33) über der Länge des Dilatatorabschnitts (3) veränderlich ist und ausgehend von dem distalen Dilatatorende (32) in proximaler Richtung zunimmt.

3. Medizinisches Gerät (1) nach Anspruch 1 oder 2, wobei der Dilatatoraußendurchmesser (33) im Bereich des distalen Dilatatorendes (32) dem Kapillaraußendurchmesser (23) entspricht und umgekehrt.

4. Medizinisches Gerät (1) nach einem der vorhergehenden Ansprüche, wobei der Dilatatorabschnitt (3) einen Kegelbereich (34) und einen Zylinderbereich (35) aufweist, wobei der Kegelbereich (34) ausgehend von dem distalen Dilatatorende (32) mit zunehmendem Dilatatoraußendurchmesser (33) proximal längserstreckt ist und in den Zylinderbereich (35) übergeht, wobei der Dilatatoraußendurchmesser (33) in dem Zylinderbereich (35) axial unveränderlich und/oder maximal ist.

5. Medizinisches Gerät (1) nach einem der vorhergehenden Ansprüche, wobei eine Länge des Kapillarabschnitts (2) zwischen 80 % und 120 %, bevorzugt zwischen 90 % und 110 %, besonders bevorzugt zwischen 95 % und 105 %, einer Länge des Dilatatorabschnitts (3) beträgt.

6. Medizinisches Gerät (1) nach einem der vorhergehenden Ansprüche, wobei der Dilatatoraußendurchmesser (33) wenigstens 120 %, bevorzugt wenigstens 150 %, besonders bevorzugt wenigstens 200 %, des Kapillaraußendurchmessers (33) beträgt.

7. Medizinisches Gerät (1) nach einem der vorhergehenden Ansprüche, wobei das proximale Kapillarende (21) und das distale Dilatatorende (32) mittels einer Fügeverbindung (C) verbunden sind und/oder wobei der Kapillarabschnitt (2) und der Dilatatorabschnitt (3) axial zusammengesteckt sind.

8. Medizinisches Gerät (1) nach einem der vorhergehenden Ansprüche, weiter aufweisend einen Griffabschnitt (5), der mit dem proximalen Dilatatorende (31) verbunden und zum manuellen Greifen durch einen Benutzer eingerichtet ist.

9. Medizinisches Gerät (1) nach einem der vorhergehenden Ansprüche, weiter aufweisend einen Fluidkonnektor (6), der proximal in das Lumen (4) mündet, wenigstens mittelbar mit dem Dilatatorabschnitt (3) verbunden ist und zur Verbindung mit einer fluidführenden Komponente eingerichtet ist.

10. Medizinisches Gerät (1) nach einem der vorhergehenden Ansprüche, weiter aufweisend eine Kanüle (7), die axial in das Lumen (4) eingeführt oder einführbar ist und deren distale Kanülenspitze (72) in eingeführtem Zustand über das distale Kapillarende (22) hinausragt.
